# EUROPEAN PATENT SPECIFICATION

(11) **EP 2 727 573 B1**
(45) Date of publication and mention of the grant of the patent: **17.06.2015**
(21) Application number: 13005174.1
(22) Date of filing: 31.10.2013
(51) Int. Cl.: A61H 23/02

(54) **A stimulation device, particularly for therapy of the human body**
Ein Stimulationsgerät, insbesondere zur Therapie des menschlichen Körpers
Un dispositif de stimulation, en particulier pour la thérapie du corps humain

(30) Priority: 02.11.2012 IT AR20120034
(43) Date of publication of application: 07.05.2014
(73) Proprietor: Sergio, Paolo, 53019 Castelnuovo Berardenga (SI) (IT)
(72) Inventor: Sergio, Paolo, 53019 Castelnuovo Berardenga (SI) (IT)
(74) Representative: Olivieri, Antonella

(56) References cited:
- WO-A1-2005/094679
- WO-A2-2007/100731
- US-A1- 2009 005 834

## Description

### Technical Field

The present invention relates to a stimulation device, particularly for the therapy of the human body.

### Background art

At the current state of art it is known the beneficial and therapeutic effects of the external stimulation of targeted points or specific regions of the body, for the treatment of different types of diseases or pains.

It is also known that some regions of the human body, such as for example the foot, represent preferred stimulation zones for their physiological connection with the internal organs and the various parts of the human body and the stimulation of which can have beneficial effects on a larger part of the body, and not just locally.

Among the most widespread stimulation techniques can certainly cite the electro-stimulation in contact with the epidermis.

The devices for electro-stimulation are constituted by electrical stimulators which are able to electrically stimulate some muscle bundles and cause muscle contraction.

The stimulation devices of known type are not free from drawbacks, including the fact that they are not generally adapted to generate a proper physiological stimulation of the interested muscles.

In fact, these electro-stimulation devices electrically stimulate the muscle bundles, determining their contraction, with no particular correlation to increased muscle activity.

In fact on the basis of physiological modes that leads to contraction and release of muscles, the use of such stimulators devices may be nullified, and even harmful for the body when electrical stimulation is generated with timing completely uncorrelated with respect to the movement of the patient.

For example, an electro-stimulator may stimulate a muscle contraction when the brain has commanded to perform a relaxation of the same muscle, or a contraction of the antagonist muscle.

In order not to cancel the stimulator effect, and then the muscle contraction, for example when the two signals are equal but in antiphase, electric intensities often very high are used, with possible contraindications for the user, since the physiological signals of muscles add up to the stimulation signal generated by the stimulation device.

Another drawback of such known stimulator devices consists in the fact that the signal of electrical stimulation generated by them consists of predetermined amplitude and frequency content, and is therefore completely uncorrelated with the instantaneous muscle activity of the user, or with the physiological demand of the muscle. Document WO-A-2005/094679 discloses the most relevant prior art.

### Disclosure of the invention

The aim of the present invention consists in the fact of realizing a stimulation device, particularly for the therapy of the human body, which overcomes drawbacks and limits of the prior art allowing to stimulate muscle contraction according to the actual physiological muscular requirements of the user.

Within this aim, an object of the present invention is to provide a stimulation device that is adapted to stimulate muscle contraction in a synchronous manner with the muscle motion of the user.

Another object of the invention consists in the fact of making a stimulation device that is able to generate a contraction muscle stimulation correlated to, in amplitude and frequency, the actual physiological muscle requirements of the user in the instant of use of the device.

Another object of the invention consists in the fact of providing a device that is easily wearable and therefore usable at any time. Another object of the invention consists in the fact of making a stimulation that does not require a direct contact between skin and stimulator element.

A further object of the invention consists in the fact of making a stimulation device which is adapted to give the wider assurances of reliability and safety in use.

Another object of the invention consists in the fact of making a stimulation device that is easy to be realized and economically competitive compared to the prior art.

The above aim, as well as the purposes mentioned and others which will become apparent hereinafter are achieved by a stimulation device according to claim 1.

### Brief description of the drawing

Further characteristics and advantages will become apparent from the description of two preferred, but not exclusive, embodiments of a stimulation device, illustrated by way of non-limiting example, with the aid of the accompanying drawings in which:
Figure 1 is a top plan view of a first embodiment of a stimulation device according to the invention, applied to an insole of a shoe;
Figure 2 is a cross section of the stimulation device illustrated in Figure 1;
Figure 3 is a schematic representation illustrating a first variant of the stimulation device illustrated in Figure 1;
Figure 4 is a schematic representation illustrating a second variant of the stimulation device illustrated in Figure 1;
Figure 5 is a schematic representation illustrating a third variant of the stimulation device illustrated in Figure 1;
Figure 6 is a schematic representation illustrating a fourth variant of the stimulation device illustrated in Figure 1;
Figure 7 is an exploded perspective view of the stimulation device according to the invention;
Figure 8 is a top plan view of a second embodiment of the stimulation device according to the invention;
Figure 9 is a graph illustrating the signal generated by the stimulation device, according to the invention, in a first mode of use;
Figure 10 is a graph illustrating the signal generated by the stimulation device, according to the invention, in a second mode of use.

### Ways of carrying out the Invention

With reference to the above figures, the stimulation device, particularly for the therapy of the human body, generally designated by the reference numerals 1 and 100 respectively in the first and second embodiment, includes stimulation means adapted to be arranged proximate to a region to be stimulated of a user.

According to the invention, the stimulation device 1, 100 includes sensor means adapted to detect a muscle motion of the user, and functionally connected to the stimulator means for their control so as to stimulate the interested region in a synchronous manner with respect to muscle motion of the user.

The sensor means may comprise at least one piezoelectric element 2 adapted to detect muscle motion. Advantageously, both stimulator means and sensor means comprise said piezoelectric element 2 in such a way that it can detect muscle motion and at the same time stimulate the interested region.

It is known that a piezoelectric element can operate in two modes.

In a first mode, also called "active mode", it provides electrical energy to the piezoelectric element, for example by applying a voltage (potential difference) to the piezoelectric element, and it reacts by deforming mechanically and then emitting mechanical vibrations.

The intensity of the vibrations is related to the amplitude of the voltage applied to the piezoelectric element; the vibration frequency is related to the frequency of the voltage applied to the piezoelectric element.

In a second mode, also referred to as "passive mode", the piezoelectric element subjected to mechanical stress, for example due to the pressure of the same, reacts by emitting electrical energy, i.e. generating a voltage. Even in this case, amplitude and frequency of the generated voltage signal are related to the intensity and frequency of the mechanical stress to which the piezoelectric element is subjected.

Depending on the type of piezoelectric element adopted, we can reach very high values of voltage, even in the order of some tens of Volts.

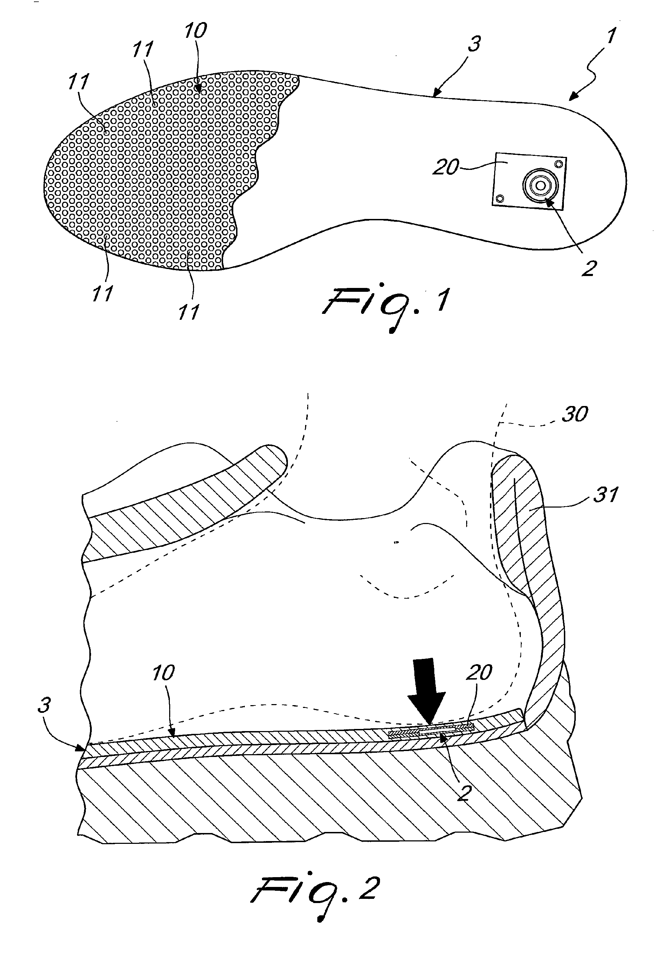

As illustrated, for example, in Figure 1, the stimulation device 1, 100 may be placed in a plantar or in an insole 3 for footwear 31. In this way, the pressure of a certain region of the foot 30, for example the heel, determines a mechanical deformation of the piezoelectric element 2, which reacts by generating a voltage signal that goes to stimulate this region of the foot.

The piezoelectric element 2 generates a voltage signal even when its mechanical deformation, for example due to the pressure of the heel, is released, for example in the phases of the gait cycle in which the heel is lifted.

The piezoelectric element 2 is advantageously placed in an electronic base 20.

In other words, the piezoelectric element 2 acts as a sensor adapted to detect muscle motion, for example related to the step, and at the same time acts as a stimulator, as it generates an electric voltage, and therefore a passage of current in the device 1, 100.

This relation "sensor/stimulator" of the piezoelectric element 2 occurs whether as a result of pressing the same that following the release of the pressure.

Furthermore, the device 1, 100 also generates a magnetic field of stimulation of the interested region. The stimulation is therefore correlated and synchronized with the motion of the same muscle. The energy generated by the stimulation device 1, 100, and especially from the piezoelectric element 2 included therein, it is always synchronized with the muscle motion of the patient, as it will be emitted precisely when the heel presses on the device 1, 100, thereby strengthening the action of the muscle.

Furthermore, the energy generated by the stimulation device 1, 100 is modulated in amplitude, and in the frequency content, with the actual needs of muscles, as very powerful muscle motions, such as for example those related to running, will generate a mechanical deformation of the piezoelectric element 2 different, in terms of amplitude and frequency content, compared to muscle motions of mild intensity, such as for example those related to walking.

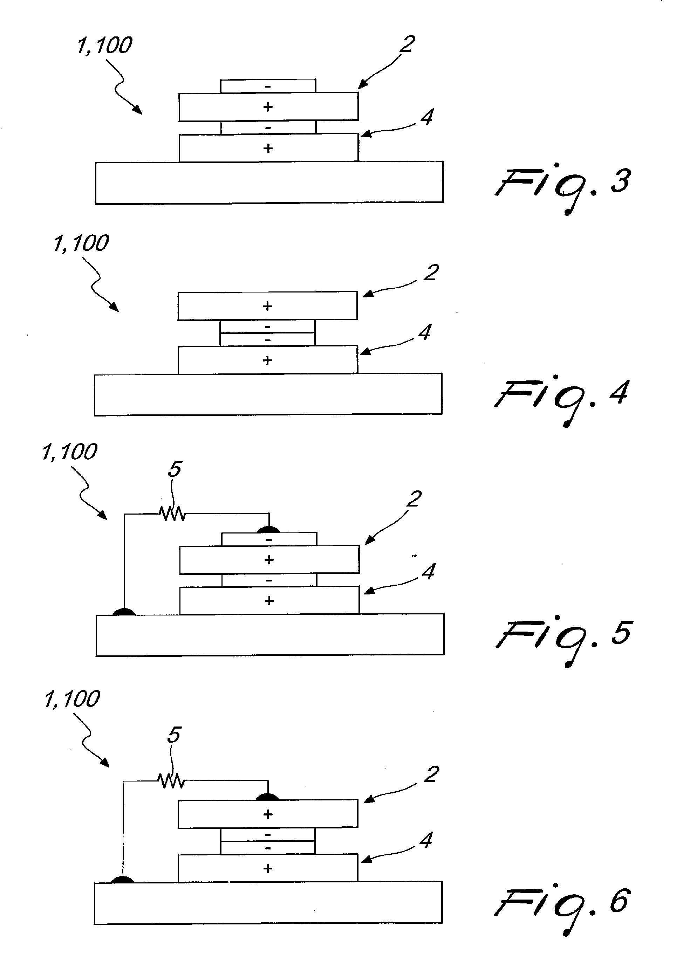

Advantageously, the stimulation device 1, 100 comprises at least two piezoelectric elements 2, 4, as illustrated in the schematic representations of figures 3 to 6, concerning four variants of the stimulation device 1, 100.

Those two piezoelectric elements 2, 4 can be connected together in series so as to raise the working voltage, or in parallel so as to raise the intensity of their working current. In both cases it is possible to predict at least one electrical resistor 5 connected to the two piezoelectric elements 2, 4 in such a manner as to define a closed loop electrical circuit.

The first variant of the stimulation device 1, 100 is illustrated in Figure 3. This Figure 3 illustrates in particular a schematic representation of two piezoelectric elements 2, 4 connected in series, i.e. so that the face with the positive polarity of the piezoelectric element 4 is in contact with the face with negative polarity of the piezoelectric element 2. In figure 3 the two piezoelectric elements 2, 4 are shown stacked one above the other. The voltage of the stimulation device 1, 100 is therefore equal to the sum of the voltages of the individual piezoelectric elements 2, 4.

The second variant of the stimulation device 1, 100 is illustrated in Figure 4. This figure 4 shows a schematic representation of two piezoelectric elements 2, 4 connected in parallel, i.e. so that the face with the negative polarity of the piezoelectric element 4 is in contact with the face with negative polarity of the piezoelectric element 2. In Figure 4, the two piezoelectric elements 2, 4 are shown stacked one above the other. In this case the electric current of the stimulation device 1, 100 is therefore equal to the sum of the electric currents of the individual piezoelectric elements 2, 4.

The third and the fourth variant of the stimulation device 1, 100 are illustrated respectively in Figures 5 and 6. These Figures 5 and 6 illustrate respectively the stimulation device 1, 100 of Figures 3 and 4, in which has been added an electric resistor 5 connected to the two piezoelectric elements 2, 4 as to define a closed loop electrical circuit.

In the open-loop solution illustrated in Figures 3 and 4 (first and second variant), there being not a real circulation of the current in the device 1, 100, the device 1, 100 is not able to generate relevant electromagnetic fields, but, just for the fact that the device 1, 100 is not fed back, it is able to generate a very high spectrum of frequency harmonics.

In the in closed loop solution illustrated in Figures 5 and 6 (third and fourth variant), the added electric resistor 5 determines a passage of electric current in the device 1, 100, determined by the same value of resistance, generating a known electromagnetic field. However, being the device 1, 100 fed back, the working frequency band of the device 1, 100 will be more limited, and the amount of emitted frequency harmonics will be more contained.

Preferably, according to a second embodiment, the stimulation means comprise at least one vibrating element 7, such as for example a vibrating micro-motor, fed directly from the voltage signal generated from the piezoelectric element 2, or from a battery 8. The mechanical vibrations generated by this vibrating element 7 generate a stimulation of mechanical type, i.e. vibratory stimulation, associated with the electro/magnetic stimulation generated from the piezoelectric element 2. This stimulation of mechanical type is synchronized and correlated in terms of amplitude and frequency content to muscle motion of the user, since the vibrating element is powered directly from the piezoelectric element 2, or, in the case of battery 8, as the power supply of the vibrating element is still modulated according to the signal detected by sensor means, i.e. from the piezoelectric element 2 itself.

Referring again to the fourth variant of the stimulation device 1, 100, illustrated in Figure 6, the device 1, 100, comprising two piezoelectric elements 2, 4 connected in parallel and connected by an electrical resistor 5, appears to be adapted to generate both an electro/magnetic stimulation and a mechanical stimulation.

In fact, the pressure of one of the two piezoelectric elements 2, 4 generates a voltage that, due to the fact that the two piezoelectric elements are connected in parallel, is applicable to the heads of the other piezoelectric element, de facto energizing it and causing the mechanical deformation, and then the vibration. In other words, in such a configuration of connection of the two piezoelectric elements 2, 4, one of them would be supplied from the other, and vice versa, and therefore able to vibrate and mechanically stimulate the interested region.

For illustrative purposes, the placement, at two opposite ends of the insole 3, of the two piezoelectric elements 2, 4, connected in parallel and connected by the resistor 5, would mean that, during the different phases of the step, the two piezoelectric elements alternate the function of sensor and electro/magnetic stimulator to the function of mechanical stimulator. For example, the pressure with the front portion of the foot of a piezoelectric element generates a voltage signal that determines an electro/magnetic stimulation of the piezoelectric element, and at the same time the power supply of the other piezoelectric element, placed for example in correspondence of the heel, which therefore mechanically vibrates and thus also mechanically stimulates the sole of the foot.

The role of the two piezoelectric elements is reversed when the element arranged in correspondence of the heel is pressed and the one in the front portion of the sole of the foot is released.

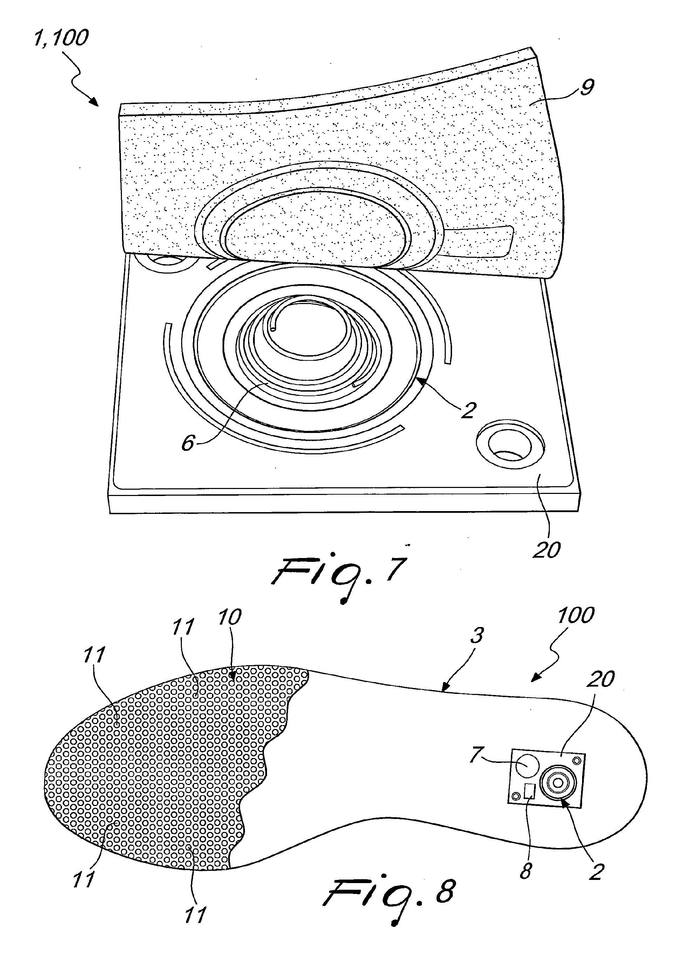

Advantageously, as illustrated in Figure 7, stimulation means may comprise elastic means 6, such as for example a spring, arranged to amplify the stimulation due to mechanical vibrations. A thin layer of flexible material 9 is applicable to the stimulation device 1, 100 to protect the piezoelectric element 2. The physical properties of the layer of flexible material 9 are such as not to affect the pressure transmission on the piezoelectric element 2.

Furthermore it is possible to provide means of calibration of the pressure sensitivity of the piezoelectric element. In particular, these calibration means can be constituted by an element of thickness, for example a disc, to be inserted between the piezoelectric element and the surface on which it rests, so as to increase the pressure acting on the piezoelectric, during its use, and thus increase the power of the electrical signal generated by it.

The stimulation device 1, 100 preferably also comprises a layer 10 of material comprising a plurality of rigid bodies 11 substantially spherical, interposed between the stimulator means and the region to be stimulated, said layer has the function of amplifying the effect of stimulation of the region to be stimulated.

The region to be stimulated is selected from the group consisting, for example, of one area of the foot, of an articulation, and of an area of the spine.

The stimulation device 1, 100 may be applied to the inside of a shoe, thanks to its small size, or as an accessory in the construction of tailored premedical, biomechanical or proprioceptive orthotics.

Alternatively, the stimulation device 1, 100 may also be housed in special tools for the treatment of different regions of the body, in a manner similar to what happens, for example, with acupuncture.

In particular, the stimulation device 1, 100 may also find applications in bracelets or watch straps, so as to allow to detect signals relating to the perceived heartbeat as mechanical pulse, at the level of the wrist of a user, and then operate a stimulation.

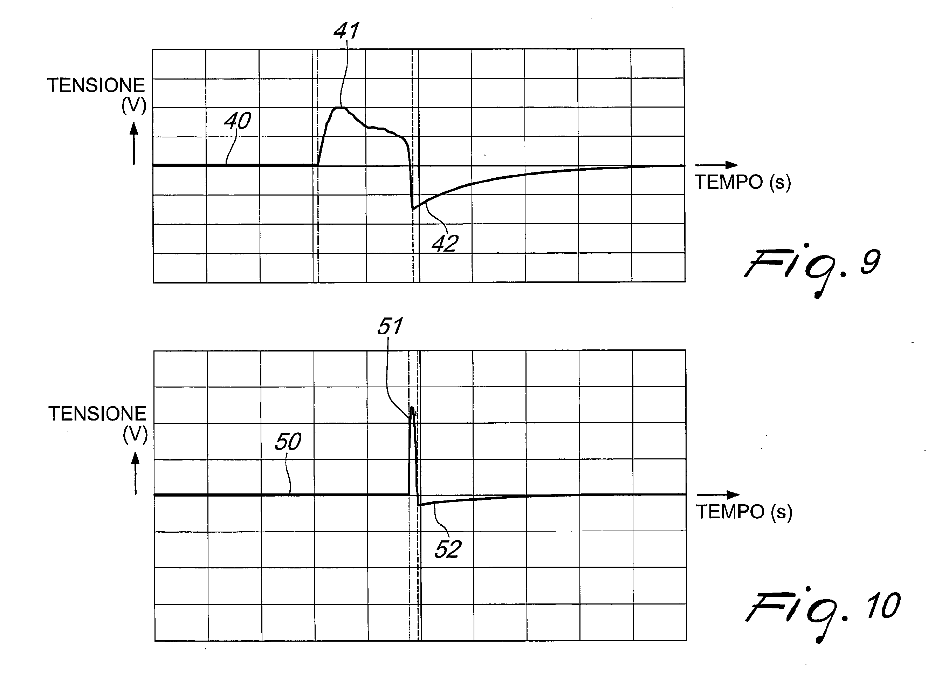

Figures 9 and 10 illustrate two examples of operation of the stimulation device 1, 100 in two different conditions of use. In both examples, reference is made to a stimulation device 1, 100 placed in an region near the sole of the foot in correspondence to the heel, for example in an insole 3.

Figure 9 illustrates the voltage signal 40 generated by the stimulation device 1, 100 subjected to a pressure of average intensity and slow dynamics. This condition is a slow walk.

The stimulation device 1, 100 outputs a positive voltage 41 high enough during the pressure, i.e. during the support of the heel, and emits a negative voltage 42 to the release of the pressure, i.e. during the lifting of the heel. The shape and the width of the voltage signal 40 corresponds to a harmonic content of the signal correlated to the intensity and to the dynamics of the step.

Figure 10 illustrates the voltage signal 50 generated by the stimulation device 1, 100 subjected to pressure of high intensity and fast dynamics. This condition represents a brisk walk or a run. The stimulation device 1, 100 still emits a positive voltage 51 high enough during the pressure, i.e. during the support of the heel, but the negative voltage 52 is virtually absent from the release of the pressure, i.e. during the lifting of the heel.

The shape and the width of the voltage signal 50 correspond to a harmonic content of the signal correlated to the intensity and to the dynamics of the step, and evidently different from that of slow walking.

It is found in practice that the stimulation device, according to the present invention, fulfills the aim as well as the intended purposes as it allows to generate stimulations through electro/magnetic fields and micro-vibrations of a broad spectrum of harmonics and synchronized with muscle motion. In fact, the stimulation device according to the invention, is adapted to generate stimulation synchronized with the user's movements, the frequency and the harmonic content of which are correlated with the muscle activity itself.

Another advantage of the stimulation device, according to the invention, consists in being adapted to treat pathologies of the painful foot, as well as all those pathologies hidden from the asymptomatic foot, such as pain in the back, hips, knees, ankles, as, even if the foot does not hurt, its incorrect supportive function determines a negative muscle adaptation by the other parts of the body.

In particular, tests were carried out on a sample of patients suffering from flat foot, hind foot valgus and atonic, which have used for a year orthotics integrating the stimulation device according to the invention. The results of these tests were compared to the results obtained from a similar sample of patients who have used the same orthotics, but non-integrating the stimulation device. The most obvious results occurred in patients presenting a general muscle weakness, which have shown significant improvements in the well-known test of baropodometric, kinesiology muscle testing and postural tests with three-dimensional scanners, showing, in particular, a greater responsiveness to kinesiological tests.

The above tests have shown that the adoption of orthotics integrating the stimulation device, according to the invention, achieves important benefits in cases of primary and secondary painful foot, asymptomatic non-functional foot, diseases and pain syndromes of the major joints, and back pain. These tests have also revealed the near absence of reactions to addiction from the user, and also the absence of effects due to overdosing of the stimulation.

Another advantage of the stimulation device, according to the invention, consists in the fact of requiring very little energy to obtain substantial benefits.

A further advantage of the stimulation device, according to the invention, consists in the fact of being usable for stimulating different region of the body.

The stimulation device thus conceived is susceptible of numerous modifications and variations, all within the inventive concept.

Moreover, all the details may be replaced with other technically equivalent elements.

In practice, the materials employed, provided they are compatible with the specific use, as well as the contingent shapes and dimensions, may be any according to requirements.

## Claims

1. A stimulation device (1, 100), particularly for therapy of the human body, comprising stimulation means adapted to be arranged proximate to a region to be stimulated of a user, sensor means that are adapted to detect a muscle motion of said user and are functionally connected to said stimulation means to control said stimulation means so as to stimulate said region synchronously with respect to said muscle motion, **characterized in that** it comprises a layer (10) of material that comprises a plurality of substantially spherical rigid bodies (11), said layer being interposed at least between said stimulation means and said region to be stimulated, said layer (10) being adapted to amplify the stimulation effect of said region to be stimulated.

2. The stimulation device (1, 100) according to the preceding claim, **characterized in that** said sensor means comprise at least one piezoelectric element (2, 4) adapted to detect said muscle motion.

3. The stimulation device (1, 100) according to claim 2, **characterized in that** said stimulation means and said sensor means comprise said at least one piezoelectric element (2, 4), so that said at least one piezoelectric element (2, 4) can detect said muscle motion and at the same time stimulate said region.

4. The stimulation device (1, 100) according to claim 3, **characterized in that** said at least one piezoelectric element (2, 4) comprises at least two piezoelectric elements (2, 4).

5. The stimulation device (1, 100) according to claim 4, **characterized in that** said at least two piezoelectric elements (2, 4) are mutually connected in series so as to raise the operating voltage of said at least two piezoelectric elements (2, 4).

6. The stimulation device (1, 100) according to claim 4, **characterized in that** said at least two piezoelectric elements (2, 4) are mutually connected in parallel so as to raise the intensity of the working current of said at least two piezoelectric elements (2, 4).

7. The stimulation device (1, 100) according to claims 5 or 6, **characterized in that** it comprises at least one electric resistor (5) connected to said at least two piezoelectric elements (2, 4) so as to form a closed-loop electric circuit.

8. The stimulation device (1, 100) according to one or more of the preceding claims, **characterized in that** said stimulation means comprise elastic means (6) adapted to amplify the stimulation.

9. The stimulation device (100) according to claims 1 or 2, **characterized in that** said stimulation means comprise at least one vibrating element (7).

10. The stimulation device (1, 100) according to one or more of the preceding claims, **characterized in that** said region to be stimulated is selected from the group constituted by a region of the sole of the foot, a joint and a region of the spine.

## Patentansprüche

1. Stimulationsvorrichtung (1, 100), insbesondere zur Therapie des menschlichen Körpers, mit Stimulationsmitteln, die geeignet sind, in unmittelbarer Nähe eines zu stimulierenden Bereichs eines Nutzens angeordnet zu werden, und Sensormitteln, die geeignet sind, eine Muskelbewegung des Nutzens zu erfassen, und die funktionell mit den Stimulationsmitteln verbunden sind zur Steuerung der Stimulationsmittel, um den Bereich synchron zu der Muskelbewegung zu stimulieren, **gekennzeichnet dadurch, dass** sie eine Schicht (10) aus Material umfaßt, das eine Vielzahl im Wesentlichen sphärischer fester Körper (11) umfasst, wobei die Schicht zumindest wischen den Stimulationsmitteln und dem zu stimulierenden Bereich angeordnet ist, wobei die Schicht (10) geeignet ist, die Stimulationswirkung des zu stimulierenden Bereichs zu verstärken.

2. Stimulationsvorrichtung (1, 100) nach Anspruch 1, **gekennzeichnet dadurch, dass** die Sensormittel zumindest ein piezoelektrisches Element (2, 4) umfassen, das zum Erfassen der Muskelbewegung geeignet ist.

3. Stimulationsvorrichtung (1, 100) nach Anspruch 2, **gekennzeichnet dadurch, dass** die Stimulationsmittel und die Sensormittel das zumindest eine piezoelektrische Element (2, 4) umfassen, sodass das zumindest eine piezoelektrische Element (2, 4) die Muskelbewegung erfassen und gleichzeitig den Bereich stimulieren kann.

4. Stimulationsvorrichtung (1, 100) nach Anspruch 3, **gekennzeichnet dadurch, dass** das zumindest eine piezoelektrische Element (2, 4) zumindest zwei piezoelektrische Elemente (2,4) umfasst.

5. Stimulationsvorrichtung (1, 100) nach Anspruch 4, **gekennzeichnet dadurch, dass** die zumindest zwei piezoelektrischen Elementen (2, 4) zueinander in Reihe geschaltet sind, um die Betriebsspannung der zumindest zwei piezoelektrischen Elemente (2,4) zu erhöhen.

6. Stimulationsvorrichtung (1, 100) nach Anspruch 4, **gekennzeichnet dadurch, dass** die zumindest zwei piezoelektrischen Elementen (2, 4) zueinander parallel geschaltet sind, um die Stärke des Betriebsstroms der zumindest zwei piezoelektrischen Element (2, 4) zu erhöhen.

7. Stimulationsvorrichtung (1. 00) nach Anspruch 5 oder 6, **gekennzeichnet dadurch, dass** sie zumindest einen elektrischen Widerstand (5) umfasst, der mit den zumindest zwei piezoelektrischen Elementen (2, 4) verbunden ist, um einen geschlossenen Stromkreis zu bilden.

8. Stimulationsvorrichtung (1, 100) nach einem oder mehreren der vorhergehenden Ansprüche, **gekennzeichnet dadurch, dass** die Stimulationsmittel elastische Mittel (6) umfassen, die geeignet sind, die Stimulation zu verstärkern.

9. Stimulationsvorrichtung (1, 100) nach Anspruch 1 oder 2, **gekennzeichnet dadurch, dass** die Stimulationsmittel zumindest ein vibrierendes Element (7) umfassen.

10. Stimulationsvorrichtung (1, 100) nach einem oder mehreren der vorhergehenden Ansprüche, **gekennzeichnet dadurch, dass** der zu stimulierende Bereich ausgewählt ist unter einem Fußsohlenbereich, einem Gelenk und einem Wirbelsäulenbereich,

## Revendications

1. Dispositif de stimulation (1, 100), particulièrement pour la thérapie du corps humain, comprenant un moyen de stimulation adapté pour être agencé à proximité d'une région à stimuler d'un utilisateur, un moyen de capteur qui est adapté pour détecter un mouvement musculaire dudit utilisateur et est fonctionnellement raccordé au dit moyen de stimulation de façon à commander ledit moyen de stimulation pour stimuler ladite région de manière synchronisée relativement au dit mouvement musculaire, **caractérisé en ce qu'**il comprend une couche (10) de matériau qui comprend une pluralité de corps rigides sensiblement sphériques (11), ladite couche étant interposée au moins entre ledit moyen de stimulation et ladite région à stimuler, ladite couche (10) étant adaptée pour amplifier l'effet de stimulation de ladite région à stimuler.

2. Dispositif de stimulation (1, 100) selon la revendication précédente, **caractérisé en ce que** ledit moyen de capteur comprend au moins un élément piézoélectrique (2, 4) adapté pour détecter ledit mouvement musculaire.

3. Dispositif de stimulation (1, 100) selon la revendication 2, **caractérisé en ce que** ledit moyen de stimulation et ledit moyen de capteur comprennent lesdits au moins un éléments piézoélectriques (2, 4), de telle sorte que lesdits au moins un éléments piézoélectriques (2, 4) puissent détecter ledit mouvement musculaire et en même temps stimuler ladite région.

4. Dispositif de stimulation (1, 100) selon la revendication 3, **caractérisé en ce que** lesdits au moins un éléments piézoélectriques (2, 4) comprennent au moins deux éléments piézoélectriques (2, 4).

5. Dispositif de stimulation (1, 100) selon la revendication 4, **caractérisé en ce que** lesdits au moins deux éléments piézoélectriques (2, 4) sont mutuellement raccordés en série pour augmenter la tension de fonctionnement desdits au moins deux éléments piézoélectriques (2, 4).

6. Dispositif de stimulation (1, 100) selon la revendication 4, **caractérisé en ce que** lesdits au moins deux éléments piézoélectriques (2, 4) sont mutuellement raccordés en parallèle de façon à augmenter l'intensité du courant de travail desdits au moins deux éléments piézoélectriques (2, 4).

7. Dispositif de stimulation (1, 100) selon la revendication 5 ou 6, **caractérisé en ce qu'**il comprend au moins une résistance électrique (5) raccordée aux dits au moins deux éléments piézoélectriques (2, 4) de façon à former un circuit électrique à boucle fermée.

8. Dispositif de stimulation (1, 100) selon l'une ou plusieurs des revendications précédentes, **caractérisé en ce que** ledit moyen de stimulation comprend un moyen élastique (6) adapté pour amplifier la stimulation.

9. Dispositif de stimulation (100) selon la revendication 1 ou 2, **caractérisé en ce que** ledit moyen de stimulation comprend au moins un élément vibrant (7).

10. Dispositif de stimulation (1, 100) selon l'une ou plusieurs des revendications précédentes, **caractérisé en ce que** ladite région à stimuler est sélectionnée dans le groupe constitué d'une région de la plante des pieds, d'une articulation et d'une région de la colonne vertébrale.
